# FASCICULE DE BREVET EUROPEEN

(11) **EP 1 199 063 B1**
(45) Date de publication et mention de la délivrance du brevet: **23.07.2003**
(21) Numéro de dépôt: 01402384.0
(22) Date de dépôt: 17.09.2001
(51) Int. Cl.: A61K 7/02, A61K 7/032

(54) **Composition de maquillage de la peau**
Schminkzusammensetzung für die Haut
Make-up composition for skin

(30) Priorité: 16.10.2000 FR 0013240
(43) Date de publication de la demande: 24.04.2002
(73) Titulaire: L'OREAL, 75008 Paris (FR)
(72) Inventeur: Piot, Bertrand, 75009 Paris (FR); Collin, Nathalie, 92330 Sceaux (FR)
(74) Mandataire: Kromer, Christophe

(56) Documents cités:
- EP-A- 1 092 416
- WO-A-00/33806
- WO-A-98/30195
- US-A- 5 261 426
- US-A- 5 993 784

## Description

La présente invention a pour objet une composition cosmétique comprenant, dans un milieu aqueux, un polymère filmogène, un tensioactif non ionique et une matière colorante pulvérulente, destinée au maquillage de la peau, y compris les lèvres. L'invention concerne également un procédé de maquillage de la peau, notamment de la peau d'être humain. La composition peut se présenter sous la forme d'eye-liner, de produit pour les lèvres ou de produit de maquillage du corps ou du visage.

Il est connu par exemple des documents FR-A-1504440 et US-A-4423031 des compositions de maquillage de la peau, en particulier les eye-liners, contenant dans un milieu aqueux des polymères filmogènes et des pigments. Les utilisatrices recherchent des compositions de maquillage présentant une bonne couvrance homogène, c'est-à-dire une composition formant un film de maquillage suffisamment coloré pour masquer la peau maquillée. Pour obtenir une couvrance homogène, les pigments doivent être bien dispersés dans le milieu aqueux sans former d'agglomérat. Pour bien disperser les pigments dans le milieu aqueux de la composition, on utilise généralement des tensioactifs dispersant. Or certains tensioactifs sont irritants pour les peaux sensibles et les compositions de maquillage contenant ces tensioactifs sont mal supportées par les utilisatrices. Ainsi, un eye-liner appliqué sur le bord des paupières peut engendrer un inconfort occulaire dû à la sensation de picotement ou de brulûres du bord des paupières provoqué par les tensioactifs irritants.

La présente invention a donc pour but de proposer une composition de maquillage de la peau, notamment du bord des paupières, contenant un polymère filmogène et des matières colorantes pulvérulentes dans un milieu aqueux, présentant de bonnes propriétés de couvrance et toléré par les peaux et/ou les yeux sensibles.

Le demandeur a constaté qu'une telle composition pouvait être obtenue en utilisant un tensioactif non ionique polycondensat tribloc polyéthylène glycol / polypropylène glycol / polyéthylène glycol. L'utilisation de ce tensioactif permet d'obtenir une bonne dispersion des matières colorantes pulvérulentes dans le milieu aqueux de la composition. La composition appliquée sur la peau forme un maquillage présentant une bonne couvrance homogène. Ce maquillage est par ailleurs bien toléré et convient donc pour le maquillage des yeux sensibles.

De façon plus précise, l'invention a pour objet une composition cosmétique de maquillage de la peau comprenant, dans un milieu aqueux cosmétiquement acceptable, un polymère filmogène, un tensioactif non ionique, ledit tensioactif non ionique étant un polycondensat tribloc polyéthylène glycol / polypropylène glycol /polyéthylène glycol, et une matière colorante pulvérulente.

L'invention a également pour objet un procédé de maquillage de la peau comprenant l'application sur la peau d'une composition telle que définie précédemment.

L'invention a aussi pour objet un procédé de maquillage du bord des paupières comprenant l'application sur le bord des paupières d'une composition telle que définie précédemment.

L'invention a encore pour objet l'utilisation d'une composition telle que définie précédemment pour obtenir un maquillage présentant une bonne couvrance et/ou toléré par les peaux et/ou les yeux sensibles.

L'invention a pour autre objet l'utilisation d'un tensioactif non ionique, ledit tensioactif non ionique étant un polycondensat tribloc polyéthylène glycol / polypropylène glycol / polyéthylène glycol, un polymère filmogène, et une matière colorante pulvérulente, dans une composition cosmétique de maquillage de la peau comprenant un milieu aqueux cosmétiquement acceptable, pour obtenir un maquillage présentant une bonne couvrance et/ou toléré par les peaux et/ou les yeux sensibles.

Par "milieu aqueux cosmétiquement acceptable", on entend un milieu compatible avec les matières kératiniques, en particulier la peau.

Le milieu aqueux de la composition peut comprendre également un solvant organique miscible à l'eau, comme par exemple des monoalcools en C₂-C₆ tels que l'éthanol, l'isopropanol. Le solvant organique miscible à l'eau, tels que les monoalcools en C₂-C₆, peut être présent en une teneur allant de 0,1 % à 20 % en poids, par rapport au poids total de la composition.

Par "polymère filmogène", on entend un polymère apte à former à lui seul ou en présence d'agent auxiliaire de filmification, un film continu et adhérent sur un support, notamment sur la peau.

Le polymère filmogène présent dans la composition selon l'invention peut être choisi parmi :
- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polyesters, notamment les polymères polyester et/ou polyesteramide anioniques, notamment dispersibles dans l'eau, comprenant des monomères portant une fonction : -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut citer en particulier les polymères décrits dans les documents US-3,734,874 ; US-4,233,196 ; US-4,304,901. Avantageusement, on choisit des polymères polyesters filmogènes à base d'au moins un acide dicarboxylique, d'au moins un diol et d'au moins un monomère aromatique bifonctionnel portant en outre un groupement -SO₃M tel que décrit ci-dessus ;
- les polyesters à chaîne grasse, les polyamides, et les résines époxyesters ;
- les polymères polyuréthannes, notamment les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes,
- les polymères d'origine naturelle, éventuellement modifiés, tels que :
   . les gommes arabiques, la gomme de guar, les dérivés du xanthane, la gomme de karaya ;
   . les alginates et les carraghénanes ;
   . les glycoaminoglycanes, l'acide hyaluronique et ses dérivés
   . la résine shellac, la gomme de sandaraque, les dammars, les élémis, les copals ;
   . l'acide désoxyribonucléïque ;
   . les muccopolysaccharides tels que l'acide hyaluronique, les chondroïtines sulfate ;
et leurs mélanges.

Le polymère filmogène peut être solubilisé ou dispersé dans le milieu aqueux de la composition.

Par "solubilisé dans le milieu aqueux", on entend un polymère qui peut être soluble dans l'eau ou dans le mélange d'eau et de solvant tel que défini précédemment.

Par "'dispersé dans le milieu aqueux", on entend un polymère insoluble dans l'eau ou le mélange d'eau et de solvants tels que définis précédemment, se présentant sous la forme de particules solides en dispersion dans le milieu aqueux. De telles dispersions peuvent être un latex, c'est-à-dire une dispersion obtenue par polymérisation en émulsion, ou bien encore un pseudolatex, c'est-à-dire une dispersion obtenue par mise en dispersion du polymère déjà synthétisé. Les techniques de préparation de ces dispersions sont bien connues de l'homme du métier.

Le polymère filmogène peut être présent dans la composition selon l'invention en une teneur allant de 0,5 % à 20 % en poids, par rapport au poids total de la composition, et de préférence allant de 1 % à 15 % en poids.

Le tensioactif non ionique présent dans la composition selon l'invention est polycondensat tribloc polyéthylène glycol / polypropylène glycol / polyéthylène glycol. Ce polycondensat tribloc a par exemple la structure chimique suivante :

H-(O-CH₂-CH₂)ₐ-(O-CH(CH₃)-CH₂)b-(O-CH₂-CH₂)ₐ-OH,

formule dans laquelle a va de 2 à 120, et b va de 1 à 100.

Le tensioactif non ionique a de préférence un poids moléculaire moyen en poids allant de 1000 à 15000, et de mieux allant de 2000 à 13000.

Avantageusement, ledit tensioactif a une température de trouble, à 10 g/l en eau distillée, supérieure ou égale à 20 °C, de préférence supérieure ou égale à 60 °C. La température de trouble est mesurée selon la norme ISO 1065.

Comme tensioactif non ionique utilisable selon l'invention, on peut citer les polycondensats tribloc polyéthylène glycol / polypropylène glycol / polyéthylène glycol vendus sous les dénominations "SYNPERONIC" comme les "SYNPERONIC PE/L44" et "SYNPERONIC PE/F127" par la société ICI.

Le tensioactif non ionique peut être présent dans la composition selon l'invention en une quantité efficace pour disperser les matières colorantes pulvérulents dans le milieu aqueux. La teneur en tensioactif non ionique peut aller de 0,1 % à 15 % en poids, par rapport au poids total de la composition, et de préférence de 1 % à 10 % en poids.

La composition de l'invention comprend une ou plusieurs matières colorantes pulvérulentes pouvant être choisies parmi les pigments, les nacres, habituellement utilisés dans les compositions cosmétiques et leurs mélanges.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La matière colorante pulvérulente peut être présente dans la composition selon l'invention en une teneur allant de 0,1 % à 40 % en poids, par rapport au poids total de la composition, et de préférence de 0,5 % à 30 % en poids.

La composition selon l'invention peut contenir, en outre, au moins un glycol pour permettre un bon mouillage des pigments, c'est-à-dire faciliter leur mise en oeuvre et leur dispersion homogène (absence d'agglomérat) dans le milieu aqueux de la composition. Le glycol permet également de plastifier le film de polymère (de rendre plus souple le film) ; il permet aussi un bon mouillage de la peau facilitant l'étalement de la composition sur la peau. Dans la présente demande, on entend par glycol un diol comprenant de 2 à 8, et de préférence de 2 à 4, atomes de carbone.

Le glycol peut être choisi parmi le propylène glycol, l'éthylène glycol, le 1,3-butylène glycol, le dipropylène glycol.

Le glycol peut être présent dans la composition en une teneur allant de 0,1 % à 30 % en poids, par rapport au poids total de la composition, et de préférence de 5 % à 20 % en poids.

Dans la composition selon l'invention, l'eau peut être présente en une teneur allant de 10 % à 90 % en poids, par rapport au poids total de la composition, et de préférence de 30 % à 50 % en poids.

La composition selon l'invention peut comprendre des charges qui peuvent être choisies parmi celles bien connues de l'homme du métier et couramment utilisées dans les compositions cosmétiques. Comme charge, on peut notamment utiliser :
- le talc qui est un silicate de magnésium hydraté utilisé sous forme de particules généralement inférieures à 40 microns,
- les micas qui sont des aluminosilicates de compositions variées se présentant sous la forme d'écailles ayant des dimensions de 2 à 200 microns, de préférence de 5 à 70 microns et une épaisseur comprise entre 0,1 à 5 microns, de préférence de 0,2 à 3 microns, ces micas pouvant être d'origine naturelle telle que la muscovite la margarite, la roscoelithe, la lipidolithe, la biotite ou d'origine synthétique,
- l'amidon en particulier l'amidon de riz,
- le kaolin qui est un silicate d'aluminium hydraté qui se présente sous la forme de particules de forme isotrope ayant des dimensions généralement inférieures à 30 microns,
- les oxydes de zinc et de titane généralement utilisés sous la forme de particules ayant des dimensions ne dépassant pas quelques microns,
- le carbonate de calcium, le carbonate ou l'hydrocarbonate de magnésium,
- la cellulose microcristalline,
- la silice,
- les poudres de polymères synthétiques tels que le polyéthylène, les polyesters (l'isophtalate ou le téréphtalate de polyéthylène), les polyamides tels que ceux vendus sous la dénomination commerciale de "Nylon" ou de "Téflon" et les poudres de silicone.

La composition selon l'invention peut comprendre au moins un additif cosmétique choisi dans le groupe formé par les agents épaississants, les conservateurs, les parfums, les colorants hydrosolubles, les vitamines, les agents hydratants, les agents adoucissants, les filtres solaires, les séquestrants, les agents alcalinisants ou acidifiants, les plastifiants.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

L'invention est illustrée plus en détail dans les exemples suivants.

### Exemple 1 :

On a préparé un eye-liner ayant la composition suivante :
- Copolymère vinyl pyrrolidone/acétate de vinyle (70/30) en solution à 50 % dans l'éthanol vendu sous la dénomination "LUVISKOL VA 73 E" par la société BASF 10 g
- Condensat oxyde d'éyhyklène/oxyde de propylène/oxyde d'éthylène (11/21/11) vendu sous la dénomination "SYNPERONIC PE/L 44" par la société ICI 6 g
- Oxyde de fer noir 20 g
- Propylène glycol 15 g
- Ethanol 7 g
- Conservateurs qs
- Eau qsp 100 g

Le liner s'applique facilement sur le bord des paupières et forme un film de maquillage présentant une bonne couvrance. Le maquillage obtenu est par ailleurs bien toléré par les personnes ayant les yeux sensibles.

### Exemple 2 :

On a préparé un eye-liner ayant la composition suivante :
- Copolymère vinyl pyrrolidone/acétate de vinyle (70/30) en solution à 50 % dans l'éthanol vendu sous la dénomination "LUVISKOL VA 73 E" par la société BASF 6 g
- Condensat oxyde d'éthylène/oxyde de propylène/oxyde d'éthylène (98/67/98) vendu sous la dénomination "SYNPERONIC PE/F 127" par la société ICI 5 g
- Oxyde de fer noir 20 g
- Propylène glycol 15 g
- Ethanol 7 g
- Silicate d'aluminium et de magnésium 1 g
- Conservateurs qs
- Eau qsp 100 g

Après application sur le bord des paupières, ce liner forme un film de maquillage présentant une bonne couvrance. Le maquillage obtenu est par également bien toléré par les personnes ayant les yeux sensibles.

## Revendications

1. Composition cosmétique de maquillage de la peau comprenant, dans un milieu aqueux cosmétiquement acceptable, un polymère filmogène, un tensioactif non ionique, ledit tensioactif non ionique étant un polycondensat tribloc polyéthylène glycol / polypropylène glycol / polyéthylène glycol, et une matière colorante pulvérulente choisie dans le groupe formé par les pigments et les nacres.

2. Composition selon la revendication 1, **caractérisée par le fait que** le tensioactif non ionique a un poids moléculaire moyen en poids allant de 1000 à 15000, et de mieux allant de 2000 à 13000.

3. Composition selon l'une quelconque des revendications précédentes, **caractérisée par le fait que** ledit tensioactif a une température de trouble, à 10 g/l en eau distillée, supérieure ou égale à 20 °C.

4. Composition selon l'une quelconque des revendications précédentes, **caractérisée par le fait que** le tensioactif non ionique est présent en une teneur allant de 0,1 % à 15 % en poids, par rapport au poids total de la composition, et de préférence de 1 % à 10 % en poids.

5. Composition selon l'une quelconque des revendications précédentes, **caractérisée par le fait que** le polymère filmogène est choisi dans le groupe formé par les polymères de kératine, les polymères de chitine ou de chitosane, les polymères cellulosiques, les polymères ou copolymères acryliques, les polymères vinyliques, les polyesters, les polyamides, les résines époxyesters, les polyuréthannes, les polymères d'origine naturelle, éventuellement modifiés, et leurs mélanges.

6. Composition selon l'une quelconque des revendications précédentes, **caractérisée par le fait que** le polymère filmogène est choisi dans le groupe formé par les polyvinylpyrrolidones, les copolymères de l'éther méthylvinylique et de l'anhydride maléique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ; l'hydroxyéthylcellulose, l'hydroxypropylcellulose, la méthylcellulose, l'éthylhydroxyéthylcellulose, la carboxyméthylcellulose, les dérivés quatemisés de la cellulose ; les polyesters, notamment les polymères polyester et/ou polyesteramide anioniques comprenant des monomères portant une fonction -SO₃M, avec M représentant un atome d'hydrogène, un ion ammonium NH₄⁺ ou un ion métallique ; les polyuréthannes-acryliques, les polyuréthannes-polyvinylpirrolidones, les polyester-polyuréthannes, les polyéther-polyuréthannes, les polyurées, les polyurée/pplyuréthannes ; les gommes arabiques, la gomme de guar, les dérivés du xanthane, la gomme de karaya ; les alginates et les carraghénates ; les glycoaminoglycanes, l'acide hyaluronique et ses dérivés ; la résine shellac, la gomme de sandaraque, les dammars, les élémis, les copals ; l'acide désoxyribonucléïque ; les muccopolysaccharides ; et leurs mélanges.

7. Composition selon l'une quelconque des revendications précédentes, **caractérisée par le fait que** le polymère filmogène est un copolymère de vinyl pyrrolidone et d'acétate de vinyle.

8. Composition selon l'une quelconque des revendications précédentes, **caractérisée par le fait que** le polymère filmogène est présent en une teneur allant de 0,5 % à 20 % en poids, par rapport au poids total de la composition, et de préférence allant de 1 % à 15 % en poids.

9. Composition selon l'une quelconque des revendications précédentes, **caractérisée par le fait que** la matière colorante pulvérulente est choisie dans le groupe formé par le dioxyde de titane, l' oxyde de zirconium, l'oxyde de cérium, I' oxyde de zinc, l'oxyde de fer, l'oxyde de chrome, le violet de manganèse, le bleu outremer, l'hydraté de chrome et le bleu ferrique, le noir de carbone, les laques à base de carmin de cochenille, de baryum, strontium, calcium, aluminium ; le mica recouvert de titane ou d'oxychlorure de bismuth, le mica titane avec des oxydes de fer, le mica titane avec notamment du bleu ferrique ou de l'oxyde de chrome ; les pigments nacrés à base d'oxychlorure de bismuth.

10. Composition selon l'une quelconque des revendications précédentes, **caractérisée par le fait que** la matière colorante pulvérulente est présente en une teneur allant de 0,1 % à 40 % en poids, par rapport au poids total de la composition, et de préférence de 0,5 % à 30 % en poids.

11. Composition selon l'une quelconque des revendications précédentes, **caractérisée par le fait qu'**elle comprend au moins un glycol ayant de 2 à 8 atomes de carbone.

12. Composition selon la revendication 11, **caractérisée par le fait que** le glycol est choisi dans le groupe formé par le propylène glycol, l'éthylène glycol, le 1,3-butylène glycol, le dipropylène glycol.

13. Composition selon la revendication 11 ou 12, **caractérisée par le fait que** le glycol est présent en une teneur allant de 0,1 % à 30 % en poids, par rapport au poids total de la composition, et de préférence de 5 % à 20 % en poids.

14. Composition selon l'une quelconque des revendications précédentes, **caractérisée par le fait qu'**elle comprend de l'eau en une teneur allant de 10 % à 90 % en poids, par rapport au poids total de la composition.

15. Composition selon l'une quelconque des revendications précédentes, **caractérisée par le fait qu'**elle comprend au moins un monoalcool en C₂-C₆.

16. Composition selon la revendication 15, **caractérisée par le fait que** le monoalcool en C₂-C₆ est présent en une teneur allant de 0,1 % à 20 % en poids, par rapport au poids total de la composition

17. Composition selon l'une quelconque des revendications précédentes, **caractérisée par le fait qu'**elle comprend un additif cosmétique choisi dans le groupe formé par les charges, les agents épaississants, les conservateurs, les parfums, les colorants hydrosolubles, les vitamines, les agents hydratants, les agents adoucissants, les filtres solaires, les séquestrants, les agents alcalinisants ou acidifiants, les plastifiants.

18. Composition selon l'une quelconque des revendications précédentes, **caractérisée par** le fait quelle se présente sous la forme d'un eye-liner ou d'un produit de maquillage du corps ou du visage, de produit pour les lèvres.

19. Eye-liner comprenant, dans un milieu aqueux cosmétiquement acceptable, un polymère filmogène, un tensioactif non ionique, ledit tensioactif non ionique étant un polycondensat tribloc polyéthylène glycol / polypropylène glycol / polyéthylène glycol, et une matière colorante pulvérulente choisie dans le groupe formé par les pigments et les nacres.

20. Procédé de maquillage de la peau comprenant l'application sur la peau d'une composition selon l'une quelconque des revendications précédentes.

21. Procédé de maquillage du bord des paupières comprenant l'application sur le bord des paupières d'une composition selon l'une quelconque des revendications 1 à 19.

22. Utilisation d'une composition selon l'une quelconque des revendications 1 à 19 pour obtenir un maquillage présentant une bonne couvrance et/ou toléré par les peaux et/ou les yeux sensibles.

23. Utilisation d'un polymère filmogène, d'un tensioactif non ionique, ledit tensioactif non ionique étant un polycondensat tribloc polyéthylène glycol / polypropylène glycol / polyéthylène glycol, et d'une matière colorante pulvérulente, dans une composition cosmétique de maquillage de la peau comprenant un milieu aqueux cosmétiquement acceptable, pour obtenir un maquillage présentant une bonne couvrance et/ou toléré par les peaux et/ou les yeux sensibles.

## Patentansprüche

1. Kosmetische Zusammensetzung zum Schminken der Haut, die in einem kosmetisch akzeptablen Medium ein filmbildendes Polymer, einen nichtionischen grenzflächenaktiven Stoff, wobei der nichtionische grenzflächenaktive Stoff ein Dreiblock-Polykondensat Polyethylenglykol/Polypropylenglykol/Polyethylenglykol ist, und ein Farbmittel in Pulverform enthält, das unter den Pigmenten und Perlglanzpigmenten ausgewählt ist.

2. Zusammensetzung nach Anspruch 1, **dadurch gekennzeichnet, dass** der nichtionische grenzflächenaktive Stoff ein Gewichtsmittel der Molmasse im Bereich von 1000 bis 15000 und besser von 2000 bis 13000 besitzt.

3. Zusammensetzung nach einem der vorhergehenden Ansprüche, **dadurch gekennzeichnet**, das der grenzflächenaktive Stoff bei 10 g/l destilliertem Wasser eine Trübungstemperatur von mindestens 20 °C aufweist.

4. Zusammensetzung nach einem der vorhergehenden Ansprüche, **dadurch gekennzeichnet, dass** der nichtionische grenzflächenaktive Stoff in einer Menge von 0,1 bis 15 Gew.-%, bezogen auf das Gesamtgewicht der Zusammensetzung, und vorzugsweise 1 bis 10 Gew.-% vorliegt.

5. Zusammensetzung nach einem der vorhergehenden Ansprüche, **dadurch gekennzeichnet, dass** das filmbildende Polymer unter den Keratinpolymeren, Chitinpolymeren, Chitosanpolymeren, Cellulosepolymeren, Acrylpolymeren, Acrylcopolymeren, Vinylpolymeren, Polyestern, Polyamiden, Epoxyestern, Polyurethanen, Polymeren natürlicher Herkunft, die gegebenenfalls modifiziert sind, und deren Gemischen ausgewählt ist.

6. Zusammensetzung nach einem der vorhergehenden Ansprüche, **dadurch gekennzeichnet, dass** das filmbildende Polymer unter den Polyvinylpyrrolidonen, Copolymeren von Methylvinylether und Maleinsäureanhydrid, dem Copolymer von Vinylacetat und Crotonsäure, Copolymeren von Vinylpyrrolidon und Vinylacetat, Copolymeren von Vinylpyrrolidon und Caprolactam; Polyvinylalkohol; Hydroxyethylcellulose, Hydroxypropylcellulose, Methylcellulose, Ethylhydroxyethylcellulose, Carboxymethylcellulose, quaternisierten Cellulosederivaten; Polyestern, insbesondere anionischen Polyestern und/oder Polyesteramiden, die Monomere enthalten, die eine Gruppe -SO₃M tragen, wobei M ein Wasserstoffatom, ein Ammoniumion NH₄⁺ oder ein Metallion bedeutet; Polyurethan-Acrylverbindungen, Polyurethan-Polyvinylpyrrolidonen, Polyester-Polyurethanen, Polyether-Polyurethanen, Polyharnstoffen, Polyharnstoff/Polyurethanen; Gummi arabicum, Guargummi, Xanthanderivaten, Karaya-Gummi; Alginaten, Carrageenaten; Glykosaminoglykanen, Hyaluronsäure und ihren Derivaten; Schellack, Sandarak, Dammarharzen, Elemi, Kopalen; Desoxyribonucleinsäure; Mucopolysacchariden und deren Gemischen ausgewählt ist.

7. Zusammensetzung nach einem der vorhergehenden Ansprüche, **dadurch gekennzeichnet, dass** das filmbildende Polymer ein Vinylpyrrolidon/Vinylacetat-Copolymer ist.

8. Zusammensetzung nach einem der vorhergehenden Ansprüche, **dadurch gekennzeichnet, dass** das filmbildende Polymer in einem Mengenanteil von 0,5 bis 20 Gew.-%, bezogen auf das Gesamtgewicht der Zusammensetzung, und vorzugsweise 1 bis 15 Gew.-% vorliegt.

9. Zusammensetzung nach einem der vorhergehenden Ansprüche, **dadurch gekennzeichnet, dass** das pulverförmige Farbmittel unter Titandioxid, Zirconiumoxid, Ceroxid, Zinkoxid, Eisenoxid, Chromoxid; Manganviolett, Ultramarinblau, Chromhydrat und Eisenblau, Ruß, Lacken auf der Basis von Cochenillerot, Barium, Strontium, Calcium oder Aluminium; mit Titan oder Bismutoxidchlorid überzogenem Glimmer, Titandioxid-Glimmer mit Eisenoxiden, Titandioxid-Glimmer insbesondere mit Eisenblau oder Chromoxid; und Perlglanzpigmenten auf der Basis von Bismutoxidchlorid ausgewählt sind.

10. Zusammensetzung nach einem der vorhergehenden Ansprüche, **dadurch gekennzeichnet, dass** das pulverförmige Farbmittel in einem Mengenanteil von 0,1 bis 40 Gew.-%, bezogen auf das Gesamtgewicht der Zusammensetzung, und vorzugsweise 0,5 bis 30 Gew.-% vorliegt.

11. Zusammensetzung nach einem der vorhergehenden Ansprüche, **dadurch gekennzeichnet, dass** sie mindestens ein Glykol mit 2 bis 8 Kohlenstoffatomen enthält.

12. Zusammensetzung nach Anspruch 11, **dadurch gekennzeichnet, dass** das Glykol unter Propylenglykol, Ethylenglykol, 1,3-Butylenglykol und Dipropylenglykol ausgewählt ist.

13. Zusammensetzung nach Anspruch 11 oder 12, **dadurch gekennzeichnet, dass** das Glykol in einem Mengenanteil von 0,1 bis 30 Gew.-%, bezogen auf das Gesamtgewicht der Zusammensetzung, und vorzugsweise 5 bis 20 Gew.-% vorliegt.

14. Zusammensetzung nach einem der vorhergehenden Ansprüche, **dadurch gekennzeichnet, dass** sie Wasser in einer Menge von 10 bis 90 Gew.-%, bezogen auf das Gesamtgewicht der Zusammensetzung, enthält.

15. Zusammensetzung nach einem der vorhergehenden Ansprüche, **dadurch gekennzeichnet, dass** sie mindestens einen C₂₋₆-Monoalkohol enthält.

16. Zusammensetzung nach Anspruch 15, **dadurch gekennzeichnet, dass** der C₂₋₆-Monoalkohol in einer Menge von 0,1 bis 20 Gew.-%, bezogen auf das Gesamtgewicht der Zusammensetzung, vorliegt.

17. Zusammensetzung nach einem der vorhergehenden Ansprüche, **dadurch gekennzeichnet, dass** sie einen kosmetischen Zusatzstoff enthalt, der unter den Füllstoffen, Verdickungsmitteln, Konservierungsmitteln, Parfums, wasserlöslichen Farbmitteln, Vitaminen, Hydratisierungsmitteln, reizlindernden Mitteln, Sonnenschutzfiltern, Maskierungsmitteln, Alkalisierungsmitteln oder Ansäuerungsmitteln und Weichmachern ausgewählt ist.

18. Zusammensetzung nach einem der vorhergehenden Ansprüche, **dadurch gekennzeichnet, dass** sie als Eyeliner oder Produkt zum Schminken des Körpers oder des Gesichts oder Produkt für die Lippen vorliegt.

19. Eyeliner, der in einem kosmetisch akzeptablen Medium ein filmbildendes Polymer, einen nichtionischen grenzflächenaktiven Stoff, wobei der nichtionische grenzflächenaktive Stoff ein Dreiblock-Polykondensat Polyethylenglykol/ Polypropylenglykol/ Polyethylenglykol ist, und ein Farbmittel in Pulverform enthält, das unter den Pigmenten und Perlglanzpigmenten ausgewählt ist.

20. Verfahren zum Schminken der Haut, das umfasst, eine Zusammensetzung nach einem der vorhergehenden Ansprüche auf die Haut aufzutragen.

21. Verfahren zum Schminken der Augenlidränder, das umfasst, eine Zusammensetzung nach einem der Ansprüche 1 bis 19 auf den Rand des Augenlides aufzutragen.

22. Verwendung einer Zusammensetzung nach einem der Ansprüche 1 bis 19, zur Erzeugung einer Schminke, die gut deckt und/oder von empfindlicher Haut und/oder empfindlichen Augen gut vertragen wird.

23. Verwendung eines filmbildenden Polymers, eines nichtionischen grenzflächenaktiven Stoffes, wobei der nichtionische grenzflächenaktive Stoff ein Dreiblock-Polykondensat Polyethylenglykol/Polypropylenglykol/Polyethylenglykol ist, und eines Farbmittels in Pulverform in einer kosmetischen Zusammensetzung zum Schminken der Haut, die ein kosmetisch akzeptables Medium enthält, zur Erzeugung einer Schminke, die gut deckt und/oder von empfindlicher Haut und/oder empfindlichen Augen gut vertragen wird.

## Claims

1. Make-up cosmetic composition for the skin comprising, in a cosmetically acceptable aqueous medium, a film-forming polymer, a nonionic surfactant, the said nonionic surfactant being a polyethylene glycol/polypropylene glycol/polyethylene glycol triblock polycondensate, and pulverulent colouring matter chosen from the group formed by pigments and pearlescent agents.

2. Composition according to Claim 1, **characterized in that** the nonionic surfactant has a weight-average molecular weight ranging from 1000 to 15,000, and even better ranging from 2000 to 13,000.

3. Composition according to any one of the preceding claims, **characterized in that** the said surfactant has a cloud temperature, at 10 g/l in distilled water, greater than or equal to 20°C.

4. Composition according to any one of the preceding claims, **characterized in that** the nonionic surfactant is present in an amount ranging from 0.1% to 15% by weight, relative to the total weight of the composition, and preferably from 1% to 10% by weight.

5. Composition according to any one of the preceding claims, **characterized in that** the film-forming polymer is chosen from the group formed by keratin polymers, chitin or chitosan polymers, cellulosic polymers, acrylic polymers or copolymers, vinyl polymers, polyesters, polyamides, epoxy ester resins, polyurethanes, optionally modified polymers of natural origin, and mixtures thereof.

6. Composition according to any one of the preceding claims, **characterized in that** the film-forming polymer is chosen from the group formed by polyvinylpyrrolidones, copolymers of methyl vinyl ether and maleic anhydride, the copolymer of vinyl acetate and crotonic acid, copolymers of vinylpyrrolidone and vinyl acetate; copolymers of vinylpyrrolidone and caprolactam; polyvinyl alcohol; hydroxyethyl cellulose, hydroxypropyl cellulose, methyl cellulose, ethyl hydroxyethyl cellulose, carboxymethyl cellulose, quaternized derivatives of cellulose; polyesters, in particular anionic polyester and/or polyester amide polymers comprising monomers carrying an -SO₃M functional group, with M representing a hydrogen atom, an ammonium ion NH₄⁺ or a metal ion; polyurethane-acrylics, polyurethane-polyvinylpyrrolidones, polyester-polyurethanes, polyetherpolyurethanes, polyureas, polyurea/polyurethanes; gum arabic, guar gum, xanthan derivatives, karaya gum; alginates and carrageenans; glycoaminoglycans, hyaluronic acid and its derivatives; shellac resin, sandarac gum, dammars, elemis, copals; deoxyribonucleic acid; mucopolysaccharides; and mixtures thereof.

7. Composition according to any one of the preceding claims, **characterized in that** the film-forming polymer is a copolymer of vinylpyrrolidone and vinyl acetate.

8. Composition according to any one of the preceding claims, **characterized in that** the film-forming polymer is present in an amount ranging from 0.5% to 20% by weight, relative to the total weight of the composition, and preferably ranging from 1% to 15% by weight.

9. Composition according to any one of the preceding claims, **characterized in that** the pulverulent colouring matter is chosen from the group formed by titanium dioxide, zirconium oxide, cerium oxide, zinc oxide, iron oxide, chromium oxide, manganese violet, ultramarine blue, chromium hydrate and ferric blue, carbon black, lakes based on cochineal carmine, barium, strontium, calcium or aluminium; mica coated with titanium or bismuth oxychloride, mica-titanium with iron oxides, mica-titanium with in particular ferric blue or chromium oxide; pearlescent pigments based on bismuth oxychloride.

10. Composition according to any one of the preceding claims, **characterized in that** the pulverulent colouring matter is present in an amount ranging from 0.1% to 40% by weight, relative to the total weight of the composition, and preferably from 0.5% to 30% by weight.

11. Composition according to any one of the preceding claims, **characterized in that** it comprises at least one glycol having from 2 to 8 carbon atoms.

12. Composition according to Claim 11, **characterized in that** the glycol is chosen from the group formed by propylene glycol, ethylene glycol, 1,3-butylene glycol and dipropylene glycol.

13. Composition according to Claim 11 or 12, **characterized in that** the glycol is present in an amount ranging from 0.1% to 30% by weight, relative to the total weight of the composition, and preferably from 5% to 20% by weight.

14. Composition according to any one of the preceding claims, **characterized in that** it comprises water in an amount ranging from 10% to 90% by weight, relative to the total weight of the composition.

15. Composition according to any one of the preceding claims, **characterized in that** it comprises at least one C₂-C₆ monoalcohol.

16. Composition according to Claim 15, **characterized in that** the C₂-C₆ monoalcohol is present in an amount ranging from 0.1% to 20% by weight, relative to the total weight of the composition.

17. Composition according to any one of the preceding claims, **characterized in that** it comprises a cosmetic additive chosen from the group formed by fillers, thickening agents, preservatives, perfumes, water-soluble colorants, vitamins, moisturizing agents, emollients, sunscreens, sequestrants, alkalinizing or acidifying agents, plasticizers.

18. Composition according to any one of the preceding claims, **characterized in that** it is provided in the form of an eyeliner or of a make-up product for the body or the face, or of a product for the lips.

19. Eyeliner comprising, in a cosmetically acceptable aqueous medium, a film-forming polymer, a nonionic surfactant, the said nonionic surfactant being a polyethylene glycol/polypropylene glycol/polyethylene glycol triblock polycondensate, and pulverulent colouring matter chosen from the group formed by pigments and pearlescent agents.

20. Method for the application of make-up to the skin comprising the application, to the skin, of a composition according to any one of the preceding claims.

21. Method for the application of make-up to the edge of the eyelids comprising the application, to the edge of the eyelids, of a composition according to any one of Claims 1 to 19.

22. Use of a composition according to any one of Claims 1 to 19, for obtaining a make-up having good covering power and/or which is tolerated by sensitive skins and/or eyes.

23. Use of a film-forming polymer, of a nonionic surfactant, the said nonionic surfactant being a polyethylene glycol/polypropylene glycol/polyethylene glycol triblock polycondensate, and of pulverulent colouring matter, in a make-up cosmetic composition for the skin comprising a cosmetically acceptable aqueous medium, for obtaining a make-up having good covering power and/or which is tolerated by sensitive skins and/or eyes.
